(19) **Europäisches Patentamt**

**European Patent Office**

**Office européen des brevets**

(11) **EP 0 551 820 B1**

(12) **EUROPÄISCHE PATENTSCHRIFT**

(45) Veröffentlichungstag und Bekanntmachung des Hinweises auf die Patenterteilung:
**03.11.1999 Patentblatt 1999/44**

(51) Int. Cl.$^6$: **A61K 9/50**, A61K 9/10

(21) Anmeldenummer: **93100079.8**

(22) Anmeldetag: **05.01.1993**

(54) **Geschmacksmaskierte pharmazeutische Mittel**

Taste-masked pharmaceutical compositions

Compositions pharmaceutiques masquant le mauvais goût

(84) Benannte Vertragsstaaten:
**AT BE CH DE DK ES FR GB GR IE IT LI LU MC NL PT SE**

(30) Priorität: **15.01.1992 DE 4200821**

(43) Veröffentlichungstag der Anmeldung:
**21.07.1993 Patentblatt 1993/29**

(73) Patentinhaber: **BAYER AG**
**51368 Leverkusen (DE)**

(72) Erfinder:
- **Pöllinger, Norbert, Dr.**
  **W-5068 Odenthal 3 (DE)**
- **Michaelis, Johannes, Dr.**
  **W-5000 Köln 80 (DE)**

- **Benke, Klaus, Dr.**
  **Kyoto-City (JP)**
- **Rupp, Roland, Dr.**
  **W-5653 Leichlingen 2 (DE)**
- **Bücheler, Manfred, Dipl.-Ing.**
  **W-5063 Overath 2 (DE)**

(56) Entgegenhaltungen:
**EP-A- 0 189 114      EP-A- 0 295 941**
**EP-A- 0 312 340      EP-A- 0 403 959**
**EP-A- 0 409 254**

Bemerkungen:
Die Akte enthält technische Angaben, die nach dem Eingang der Anmeldung eingereicht wurden und die nicht in dieser Patentschrift enthalten sind.

Printed by Xerox (UK) Business Services
2.16.7/3.6

## Beschreibung

Die Erfindung betrifft geschmacksmaskierte pharmazeutische Mittel zur peroralen Anwendung, deren Herstellung und deren Verwendung als Arzneimittel.

Mit Hilfe der erfindungsgemäßen neuen Arzneizubereitungen wird es ermöglicht, daß pharmazeutische Wirkstoffe mit sehr unangenehmen organoleptischen Eigenschaften, wie z.B. sehr schlechtem Geschmack, auch in flüssiger Form verabreicht werden können.

Besonders älteren Menschen sowie schluckbehinderten Patienten bereitet die Einnahme größerformatiger Tabletten häufig erhebliche Schwierigkeiten; auch für Kinder sind großformatige Tabletten ungeeignet.

Feste Arzneiformen wie beispielsweise Lacktabletten haben zwar den Vorteil, daß der evtl. unangenehme Eigengeschmack des Wirkstoffes vom Patienten nicht verspürt wird; sie haben jedoch den Nachteil, daß sie nicht teilbar sind, ohne daß der geschmacksabdeckende Lack beschädigt wird.

Eine individuelle Dosierung des Wirkstoffes ist jedoch in der Geriatrie und der Pädiatrie häufig unbedingt erforderlich und kann durch die Bereitstellung einer variabel dosierbaren Granulat- oder Saftformulierung gewährleistet werden. Daher kann die der vorliegenden Erfindung zugrundeliegende Aufgabe nicht durch bisher bekannt gewordene Formulierungen, z.B. aus EP-A-230881 gelöst werden.

Deshalb ist es erforderlich, individuell dosierbare Arzneiformen auch von unangenehm schmeckenden Wirkstoffen zur peroralen Anwendung zu schaffen; auch die Anwendung als Pulver oder Granulat zur direkten Anwendung wäre vorteilhaft.

Die Zubereitung einer flüssigen Arzneiform, etwa durch Zerstoßen einer Tablette und Auflösen in Wasser, ist aufgrund des extrem schlechten und lange anhaltenden Bittergeschmacks zahlreicher antimikrobieller Wirkstoffe ohne spezielle geschmacksabdeckende Maßnahmen nicht möglich. Durch den außerordentlich unangenehmen Geschmack ist eine erhebliche Störung der Patienten-Compliance zu erwarten. Eine bloße Alomatisierung von Wirkstoff-Lösungen und -Suspensionen ist häufig selbst dann nicht ausreichend, wenn Aromen verwendet werden, die bestimmte Geschmacksrichtungen spezifisch überdecken sollen.

Zu den besonders unangenehm schmeckenden Wirkstoffen gehören aus der Gruppe der antimikrobiellen Mittel die Gyrasehemmer, insbesondere solche vom Typ der Naphthyridon- und Chinolon- Carbonsäuren, ganz besonders Ciprofloxacin, Norfloxacin, Ofloxacin und Enoxacin.

Neben einer vollständigen Geschmacksabdeckung ist von vielen Wirkstoffen unbedingt eine rasche und vollständige Freisetzung zu fordern, damit eine den Tabletten äquivalente Bioverfügbarkeit gewährleistet werden kann. Dies ist deshalb problematisch, weil es für zahlreiche Wirkstoffe ein Resorptionsfenster im oberen Dünndarm gibt und die Resorption in tieferen Darmabschnitten stark reduziert ist. (S. Harder, U. Fuhr, D. Beermann, A.H. Staib, Br. J. Clin. Pharmac. 30, 35 (1990)). Es sind auch bei älteren Menschen häufig auftretende Abweichungen des Magen-pH in Richtung eines hypaciden Milieus zu berücksichtigen. Das bedeutet, daß auch im schwach sauren Milieu- etwa bei pH 4,5 - eine rasche Auflösung des Wirkstoffs gewährleistet sein muß.

Die Mikroverkapselung ist an sich eine weitverbreitete Technologie, die nicht nur in der Pharmazie Anwendung findet (P.B. Deasy; Microencapsulation and related Drug Processes; M. Dekker Inc., N.Y. & Basel, 1984).

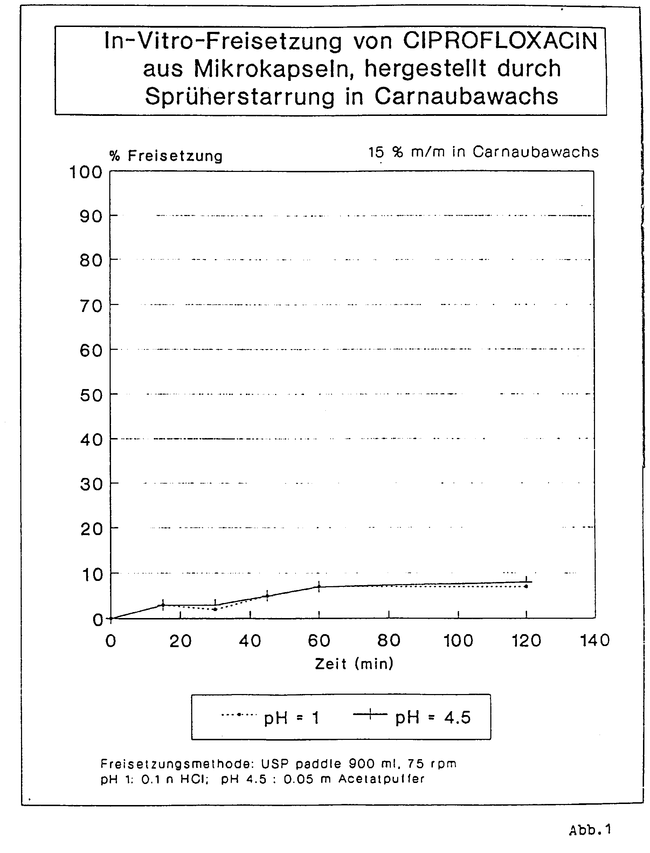

Auf pharmazeutischem Gebiet wird die Mikroverkapselung häufig angewandt, wenn eine retardierte Freisetzung von Wirkstoffen gewünscht ist. Solchermaßen hergestellte Mikrokapseln können z.B. intramusculär appliziert werden; bioabbaubare Polymere sind in der Lage, die Freisetzung der aktiven Substanz über Tage bis hin zu Wochen und Monaten zu steuern. Auch für peroral zu verabreichende Produkte ist die Mikroverkapselung mit wasserunlöslichen Lacken ein häufig eingesetztes Verfahren zur Protrahierung der Wirkstofffreisetzung, aber auch zur Geschmacksmaskierung. Die Einbettung in Wachs-Matrizes führt bekanntermaßen zu einer Geschmacksmaskierung. Aus der EP-A-273 890 ist die Geschmacksmaskierung schlecht schmeckender Arzneistoffe durch die Inkorporierung in Mikrokapseln auf der Basis von Carnauba-Wachs, Bienenwachs und Ethylcellulose oder Kombinationen hiervon bekannt geworden. Die Einbettung von z.B. Ciprofloxacin oder Ciprofloxacin-Salzen nach dem beschriebenen Verfahren führt jedoch nicht zu der erforderlichen raschen Wirkstoff-Freisetzung. Diese Art der Mikroverkapselung ist für die angestrebte perorale flüssige Zubereitung der beanspruchten Arzneistoffe nicht anwendbar. (Abb. 1).

Die EP-A-101 418 beschreibt die Mikroverkapselung von Wirkstoffen mit Kohlenwasserstoffen bzw. Kohlenwasserstoff- verwandten Substanzen mit dem Ziel einer gesteuerten Freisetzung, Geschmacksmaskierung und Wirkstoffstabilisierung. Auch mit diesen Systemen ist die rasche Freisetzung nicht erreichbar. Derartige Systeme beschreibt auch die GB-A 2 122 490; die Freisetzung der Wirkstoffe erfolgt verzögert.

Aus der DE-A-3 815 562 ist ein Verfahren zur Geschmacksmaskierung von Pharmazeutika bekannt geworden, das auf der Herstellung einer dreischichtigen Umhüllung von Arzneistoffen basiert. Die Hülle besteht aus Fett bzw. Fett und Polymer.

Aus der DE-A-3 816 464 ist ein weiteres Geschmacksmaskierungsverfahren für schlecht schmeckende Alzneistoffe bekannt geworden, das ebenfalls auf der Verwendung von Lipiden basiert. Auch das US-Patent 4 764 375 beschreibt ein Verfahren zur Geschmacksmaskierung basierend auf der Einbettung des Wirkstoffs in eine Mischung von Lipiden.

Auch aus der EP-A-0 189 114 sind Tablettenformulierungen von Chinolonen, insbesondere Norfloxacin, bekannt geworden. Hierbei handelt es sich jedoch um zur Direkttablettierung (im Gegensatz zur Tablettierung von aus Feuchtgranulation erhaltenen Mischungspartikeln) geeignete Zubereitungen. Die der vorlie-genden Erfindung zugrundeliegende Aufgabe kann damit nicht gelöst werden.

Die EP-A-0 409 254 beschreibt die Herstellung von Granulaten/Mikrokapseln, die neben der Geschmacksmaskierung gleichzeitig eine schnelle Wirkstoff-freisetzung sicherstellen sollen. Die Geschmacksmaskierung erfolgt mit Ethylcellulose-Lack. Allerdings müssen gemäß der Lehre dieses Standes der Technik die Kerne zwingend ein Sprengmittel enthalten, und zwar im Bereich von 35 - 70 %, bezogen auf das Endgewicht, um das Ziel der schnellen Freisetzung des Wirkstoffes zu erreichen. Bei Sprengmittelmengen unter 35 % erfolgt keine schnelle, sondern retardierte Freisetzung des Wirkstoffes. Im Vergleich zur vorliegenden Erfindung hat diese Darreichungsform den Nachteil, daß bei gleichem Partikelvolumen weniger Wirkstoff eingebracht werden kann (maximal 40 % bezogen auf das Endgewicht) als bei den erfindungsgemäß verwendeten Partikeln.

Die Erkenntnis, daß Lacke auf Basis Poly(meth)acrylsäure sowie deren Aminoalkylester und/oder Alkylester und Ethylcellulose zum Zweck der Geschmacksmaskierung eingesetzt werden können, ist aus dem Stand der Technik bekannt. In diesem Zusammenhang lehrt die EP-A-0 403 959 den Zusatz von wasserunlöslichen lipophilen Emulgatoren zu derartigen Lack-Dispersionen mit dem Ziel, die Verarbeitbarkeit dieser Dispersionen zu verbessern, also die Klebetendenz während des Lackierprozesses zu vermindern.

Auch die EP-A-0 312 340 ist im Zusammenhang mit der Auswahl der Lack-Komponente zu sehen. Sie beschreibt Retardformulierungen schlecht wasserlöslicher Wirksubstanzen unter Zugabe des Hilfsstoffes Carbomer. Im Gegensatz dazu lehrt die vorliegende Erfindung schnell freisetzende Formulierungen, bei denen zur Herstellung der Lackhülle solche Lacke Verwendung finden, die gemäß Stand der Technik zur Herstellung von Retardformulierungen bekannt sind, die mit der am schlechtesten wasserlöslichen Wirkstofform (Basisform) zu den schnellsten Freisetzungsraten bei gleichzeitiger Geschmacksmaskierung führt. Dieses Eigenschaftsprofil der erfindungsgemäßen Zubereitungen ist aus dem Stand der Technik nicht herleitbar.

Die EP-A-0 295 941 beschreibt oral applizierbare flüssige Suspensionen mit einer nicht-wäßrigen Ölgrundlage, wobei der suspendierte Wirkstoff verzögert freigesetzt wird. Im Gegensatz dazu werden gemäß der vorliegenden Erfindung schnell freisetzende Formulierungen beschrieben.

EP-378 137 beschreibt in Wasser dispergierbare Arzneizubereitungen, die es ermöglichen, Wirkstoffe mit organoleptisch ungünstigen Eigenschaften in flüssiger Form oral zu applizieren. Der Wirkstoff wird zunächst auf Zuckerkügelchen aufgebracht, welche in der Folge mit einer Filmschicht versehen werden. Als Arzneistoffe werden relativ niedrigdosierte Substanzen wie Pinaverium-Bromid, Metoclopramid und Salbutamol genannt. Als Polymere werden wasserunlösliche Substanzen wie Schellack und Ethylcellulose verwendet; sie werden kombiniert mit Substanzen, die unterhalb pH 5 löslich sind. Als Beispiel hierfür wird ®Eudragit E 12,5 (Röhm, Darmstadt) genannt. Der Wirkstoff soll in künstlichem Magensaft mit pH 1,2 rasch freigesetzt werden. Für ein hypacides Magenmilieu wie z.B. pH 4,5 und eine hohe Wirkstoffdosis ist diese Formulierung jedoch ungeeignet.

EP-A-212 641 beschreibt geschmacksmaskierende Zusammensetzungen, bestehend aus einer Arzneimittel-Polymermatrix, die den Wirkstoff enthält. Anmeldungsgemäß handelt es sich bei dem verwendeten Polymeren um ein Copolymer aus Methacrylsäure und deren Methylester (®Eudragit S 100). Die Matrix dissoziiert in einem Medium mit einem pH-Wert keiner 4, wobei der aktive Wirkstoff in dieses Medium freigesetzt wird.

®Eudragit S 100 dient entsprechend der Herstellerinformation der Fa. Röhm, Darmstadt, zur Herstellung magensaftresistenter, darmsaftlöslicher Lacke. Für die Geschmacksmaskierung von Wirkstoffen und die dafür geforderte rasche und vollständige Freisetzung bei pH-Werten von 1-4,5 ist ein magensaftresistenter Lack nicht geeignet.

Die Mikroverkapselung von Norfloxacin zum Zwecke der Reduzierung von Nebenwirkungen wie Magenirritationen und Hämorrhagien wird von Esmat E. Zein-El-Dien beschrieben (Pharm. Ind. 53, 87 (1991)). Als Überzugsmaterial wird wasserlösliche Methylcellulose eingesetzt; wasserunlösliche Filmbildner werden nicht erwähnt.

Die für die beanspruchten antimikrobiellen Mittel gewünschte vollständige Geschmacksmaskierung in Verbindung mit einer raschen Freisetzung ist mit den Verfahren des bisher allgemein bekannt gewordenen Standes der Technik nicht zu realisieren.

Im allgemeinen handelt es sich bei antimikrobiell wirksamen Substanzen um Strukturen, die saure oder basische funktionelle Gruppen oder gleichzeitig z.B. Carbonsäuregruppen und Amine im Molekül (Betaine) enthalten. Üblicherweise setzt man von diesen Wirkstoffen eine wasserlösliche bzw. die am besten wasserlösliche Form ein, um eine schnelle Wirkstoffreisetzung zu gewährleisten. Bei organischen Carbonsäuren sind dies im allgemeinen deren Alkali- oder Erdalkali-Salze, bei Betainen deren Carbonsäuresalze bzw. saure Salze (z.B. Hydrochloride).

Überraschenderweise wurde nun gefunden daß die Verwendung der am schlechtesten wasserlöslichen Form von Ciprofloxacin in mit speziellen Lachen hergestellten Mikrokapseln zu optimalen Ergebnissen hinsichtlich Geschmacksmaskierung und Wirkstofffreisetzung führt. Hierbei handelt es sich im allgemeinen bei Wirkstoffen, die Carbonsäuregruppen enthalten, um diese Wirkstoffe selbst und nicht deren Salze. Bei Wirkstoffen, die basische Gruppen enthalten, handelt es sich um diese Wirkform und nicht deren Salze. Erfindungsgemäß wird das Betain selbst und nicht ein Salz verwendet. Diese Form des Wirkstoffs Ciprofloxacin wird erfindungsgemäß als "Basisform des Wirkstoffs" bezeichnet. Als Einsatzstoff kann der Wirkstoff Ciprofloxacin gegebenenfalls sowohl in Form seines Hydrates oder Anhydrates verwendet werden, die fertigen Mikrokapseln enthalten den Wirkstoff Ciprofloxacin als Anhydrat.

Definitionsgemäß enthalten die Anhydrate des Wirkstoffs Ciprofloxacin in der Basisform in der erfindungsgemäßen Mikrokapsel-Formulierung weniger als 5 %, insbesondere weniger als 3,0 % Wasser in Form von Kristallwasser oder sonstigen Wasser-Addukten.

Gegenstand der Erfindung sind somit durch Mikroverkapselung geschmacksmaskierte Wirkstoffe enthaltende pharmazeutische Mittel, wie im Anspruch 1 definiert.

Die erfindungsgemäßen Mikrokapseln werden wie folgt hergestellt:

Der Wirkstoff Ciprofloxacin-Betain (im folgenden verkürzt als "Wirkstoff" bezeichnet) wird z.B. auf an sich bekannte Weise feucht granuliert, wobei als Granulierhilfsmittel Wasser oder Alkohol/Wasser-Gemische, z.B. Ethanol/Wasser-Gemische verwendet werden können. Zur Herstellung eines Mikrogranulats durch wässrige Feuchtgranulation wird vorteilhaft die Hydratform des Wirkstoffs eingesetzt. Wird zur Herstellung des Mikrogranulats die Anhydratform der Basisform des Wirkstoffs verwendet, so ist die Feuchtgranulation vorzugsweise mit Alkohol-Wasser Gemischen durchzuführen. Das Feuchtgranulat wird getrocknet und gesiebt. Die gewünschte Korngrößenfraktion wird in den anschließenden Mikroverkapselungsprozeß eingebracht. Bevorzugte Mikrogranulate werden durch Wirbelschicht-Sprühgranulation wie in EP-A-0 163 836, DE-A-3 808 116 und EP-A-0 332 929 beschrieben hergestellt. Hierbei kann eine wäßrige Suspension des Wirkstoffs, die zusätzliche Hilfsstoffe in gelöster oder suspendierter Form enthalten kann, durch ein spezielles Verfahren direkt in ein sehr gleichmäßiges Produkt aus sphärischen Wirkstoff-Hilfsstoff-Agglomeraten überführt werden. In jedem Fall wird zur Herstellung dieser Mikrogranulate die vorstehend definierte Basisform des Wirkstoffs verwendet. Bei der Herstellung des Ciprofloxacin-Mikrogranulats mit Hilfe der Wirbelschichtsprühgranulationsmethode ist ebenfalls vorteilhafterweise mikronisiertes Ciprofloxacin-Hydrat zur Herstellung der Sprühsuspension einzusetzen. Es konnte gefunden werden, daß der Einsatz der genannten Wirkstoffqualität die Herstellung sehr feinpartikulärer wäßriger Suspensionen erlaubt, ohne daß eine zusätzliche Naßmahlung erforderlich wird.

Als Bindemittel zur Erhöhung der mechanischen Festigkeit des Mikrogranulats können Stoffe wie Acacia-Gummi, Alginsäure und Alginate, Carboxymethylcellulose, Ethylcellulose, Gelatine, Hydroxypropylcellulose, Hydroxypropylmethylcellulose, Methylcellulose, Xanthan Gummi, Pektin, Traganth, mikrokristalline Cellulose, Hydroxyethylcellulose, Ethylhydroxyethylcellulose, Natriumcarboxymethylcellulose, Polyethylenglykole, Polyvinylpyrrolidon, Polyvinylalkohol, Polyacrylsäure, Gummi Arabicum, Lactose, Stärke (Weizen-, Mais-, Kartoffel-, Reis-Stärke), Saccharose, Glucose, Mannit, Sorbit, Xylit, Stearinsäure, hydriertes Baumwollsamenöl, hydriertes Rizinusöl, Vinylpyrrolidon-Vinylacetat-Copolymerisate, Fructose, Methylhydroxyethylcellulose, Agar Agar, Carrageenan, Karaya Gum, Chitosan, Stärkehydrolysate u.a. verwendet werden. Besonders vorteilhaft ist die Verwendung von Polyvinylpyrrolidon 25 in einer Konzentration von 1-10 %, bezogen auf das Mikrogranulat.

Die erfindungsgemäßen verwendeten Mikrokapseln werden hergestellt, indem man die Mikrogranulatkerne in geeigneten Apparaturen mit der erfindungsgemäßen Lackschicht überzieht.

Auf die Mikrogranulatkerne wird eine Lackschicht aufgetragen, die zu einer vollständigen Abdeckung der Granulatoberfläche führt. Dabei ist die Lackkomposition für die Mikroverkapselung in der Weise zu wählen, daß eine ausreichende Permeabilität für wäßrige Medien und eine rasche Wirkstofffreisetzung gewährleistet ist. Durch die Zusammensetzung des Lackes und dessen Dicke wird sichergestellt, daß die Mikrokapseln erst nach der Passage des geschmacksempfindlichen Bereichs aufgelöst werden, jedoch vor der Passage des Resorptionsortes die Wirkstofffreisetzung rechtzeitig erfolgt. Nicht geeignet sind daher Lacke, die zu magensaft-resistenten Überzügen führen und die Wirkstofffreisetzung erst im Darmbereich nach der Magenpassage zulassen.

Der Einsatz wäßriger Lacksuspensionen ist aus Gründen der Umwelt- und Arbeitssicherheit zu bevorzugen.

Als Filmbildner zur Herstellung von Lacken für die Herstellung von Mikrokapseln stehen an sich eine Reihe von Substanzen wie Acacia Gum, Acrylsäure-Polymere und Copolymere (Polyacrylamide, Polyacryldextrane, Polyalkylcyanoacrylate, Polymethylmethacrylate), Agar-Agar, Agarose, Albumin, Alginsäure und Alginate, Carboxyvinyl-Polymere, Cellulose-Derivate wie Celluloseacetat, Polyamide (Nylon 6-10, Poly(adipyl-L-lysine, Polyterephthalamide, Poly(terephthaloyl L-lysine)), Poly-$\varepsilon$-caprolactam, Polydimethylsiloxan, Polyester, Poly(ethylen-vinylacetat), Polyglykolsäure, Polymilchsäure und deren Copolymere, Polyglutaminsäure, Polylysin, Polystyrol, Schellack, Xanthan Gum, anionische Polymerisate aus Methacrylsäure und Methacrylsäureestern zur Verfügung, um nur einige zu nennen.

Der Auftrag der Lackschicht kann in üblichen Coater-Apparaturen wie z.B. einem nach dem Wurster-Verfahren arbeitenden Powder-Coater erfolgen. Es ist vorteilhaft, die Konzentration der Lacksuspensionen möglichst hoch zu wählen, um den Mikroverkapselungsprozeß so ökonomisch als möglich zu gestalten.

Die erfindungsgemäßen Mittel lassen sich jedoch nur mit Mikrokapseln zubereiten, zu deren Herstellung als Filmbildner neutrale Methyl- und/oder Ethyl-Esterverbindungen der Polymethacrylsäure (®Eudragit NE 30 D, Röhm, Darmstadt), und/oder quartäre Ammoniumverbindungen der Polymethacrylsäure (®Eudragit RL 30 D, ®Eudragit RS 30 D, Röhm, Darmstadt) und Ethylcellulose (®Aquacoat, FMC Corp.) verwendet werden.

Diese Lacke, die an sich nicht wasserlöslich sind, können zur Erhöhung der Permeabilität mit wasserlöslichen Polymeren kombiniert werden, die für eine Porenbildung in der Lackschicht sorgen. Als wasserlösliche Porenbildner sind Stoffe wie Hydroxypropylcellulose, Hydroxypropylmethylcellulose, Methylcellulose, Natriumcarboxymethylcellulose, Dextran, Dextrine, Cyclodextrine, Polyethylenglykole, Polyvinylalkohole, Polyvinylpyrrolidone, Stärke und Stärkehydrolysate wie z.B. modifizierte Stärketypen (gelatinierte Stärke, STA-RX 1 500, Celutab, Maltodextrine), Zucker und Zuckeraustauschstoffe wie Mono-, Di- und Oligosaccharide, Saccharose, Fructose, Lactose, Invertzucker, Mannit, Sorbit und Xylit sowie Alginsäure und Alginate, Traganth, Pektine, Gummi Arabicum und Gelatine verwendbar. Bevorzugte Porenbildner im Sinne der Erfindung sind Hydroxypropylcellulose, Hydroxypropylmethylcellulose und Methylcellulose.

Als bevorzugte Lackkombinationen von wasserunlöslichen und wasserlöslichen Komponenten sind Mischungen von ®Eudragit NE 30 D mit Hydroxypropylmethylcellulose zu nennen. So konnte mit geeigneten Mischungen dieser Stoffe, z.B. im Verhältnis 100/20 bis 100/50, bevorzugt 100/20 bis 100/40 und besonders bevorzugt 100/40 eine optimale Geschmacksabdeckung und rasche und vollständige Freisetzung des Wirkstoffs aus Mikrokapseln im pH-Bereich von 1-4,5 erreicht werden.

Selbst andere in der Fachliteratur ausdrücklich für die Geschmacksmaskierung empfohlene Lacke wie z.B. ®Eudragit E 12,5 führten nicht zu den angestrebten Resultaten hinsichtlich Geschmacksmaskierung und Freisetzungsverhalten. So könnte angesichts der EP-A-378 137 die Verwendung von ®Eudragit E 12,5 zur Geschmacksmaskierung angezeigt erscheinen. Überraschenderweise wurde jedoch gefunden, daß z.B. die Kombination aus ®Eudragit NE 30 D mit HPMC zur besten Geschmacksmaskierung führt bei gleichzeitig guter Wirkstofffreisetzung bei pH 1 und 4,5.

Weiterhin kann für die Filmbildung der Zusatz eines Weichmachers erforderlich sein. Hierbei handelt es sich um Substanzen, die die Filmbildung erleichtern und die Elastizität und mechanische Stabilität des Lacks erhöhen.

Als Weichmacher können Substanzen wie z.B. Diethylphthalat, Acetyltributylcitrat, Glycerol, Diethylsebacat, Dimethylphthalat, Dibutylphthalat, Tributylcitrat, Butylstearat, Polyethylenglykole mit unterschiedlicher Kettenlänge, Glycerolmonostearat, Triacetin, Rizinusöl und andere native und synthetische Öle, Triethylcitrat, Acetyltriethylcitrat, 1,2-Propylenglykol, acetylierte Fettsäureglyceride und Polyoxyethylen-Polyoxypropylen-Copolymere eingesetzt werden.

Die Inkorporation von oberflächenaktiven Stoffen in die Lackhülle unterstützt zum einen die Spreitung der Lackdispersion auf den Feststoffpartikeln während des Mikroverkapselungsprozesses und führt andererseits zu einer verbesserten Benetzbarkeit der Mikrokapseln. Weiterhin kann sie zu einer Beeinflussung der Lackpermeabilität beitragen.

Als Netzmittel können Stoffe wie Natriumlaurylsulfat (USP), Polysorbat (20, 40, 60, 80, 65, 61, 85, 21), Poloxamere (Ethylenoxid-Propylenoxid-Blockcopolymere) unterschiedlichen HLB-Werts, Lecithine, Ölsäure und Ölsäure-Salze, Sorbitanester (Span 20, 40, 60, 80, 85), Propylenglycolmonostearat und -monolaurat, Glycerolmonostearat und -monooleat, Brij-Typen (Fettalkohol-PEG-ether) unterschiedlichen HLB-Werts (z.B. PEG-10-cetylether, PEG-20-oleylether etc.), Myrj-Typen (Fettsäure-PEG-ester) unterschiedlichen HLB-Werts, (z.B. PEG-40-monostearat; PEG-100-monostearat u.a.), Natriumdodecylsulfat (SDS), Dioctylnatriumsulfosuccinat (DONS), ethoxylierte Mono- und Diglyceride unterschiedlichen HLB-Werts (Tagat-Typen), Saccharose-Fettsäureester, Fettsäure-Salze (Na, K, Ca, Mg, Al etc.), ethoxylierte Triglyceride (polyoxyethyliertes Rizinusöl (40), polyoxyethyliertes hydriertes Rizinusöl (40 bzw. 60), polyoxyethylierte pflanzliche Öle), Sterole (Cholesterol, Wollwachsalkohole) in Konzentrationen von 0,001-20 %, vorzugsweise 0,1-2 % eingesetzt werden.

Um die Adhäsion bzw. das Verkleben von Partikeln während des Mikroverkapselungsprozesses zu verringern oder ganz zu vermeiden, sind Antiklebemittel zuzusetzen. Geeignete Stoffe sind z.B. Magnesiumstearat, Calciumstearat, Calciumbehenat, Talkum, kolloidale Kieselsäure, Stearinsäure, Precirol (Gemisch von Mono-, Di- und Triestern von Palmitin- und Stearinsäure mit Glycerin), Hydriertes Baumwollsamenöl, Hydriertes Rizinusöl und Polyethylenglykol unterschiedlicher Molekulargewichte.

Mengen von 0,1-90 %, besonders von 5-40 % werden vorzugsweise eingesetzt.

Weiterhin können in der Lackhülle Farbstoffe enthalten sein.

Die Mikrokapseln können mit einer Polierschicht versehen werden. Sie dient nicht nur der optischen Verschönerung, sondern stellt auch eine wichtige funktionale Einheit der erfindungsgemäßen Mikrokapseln dar: durch Auftrag einer finalen Schicht über der eigentlichen Lackschicht wird eine direkte Wechselwirkung einer beispielsweise öligen Saftkomponente mit der geschmacksabdeckenden Lackschicht verhindert. Insbesondere bei Verwendung einer wasserlöslichen oder zumindest hydrophilen Polierschicht kann ein direkter Kontakt eines öligen Saft-Trägerstoffes mit der Mikrokapsel-Lackhülle und eine durch Öl/Lack-Wechselwirkungen verursachte Freisetzungsverzögerung verhindert werden. Weiterhin wird die Klebneigung von Mikrokapseln beim Einbringen in eine wäßrige Flüssigkeit verringert.

Geeignete Poliermittel sind Polyethylenglykole unterschiedlichen Molekulargewichts oder Mischungen derselben, Talkum, Tenside (Brij-Typen, Myrj-Typen, Glycerolmonostearat, Poloxamere), Fettalkohole (Stearyl-, Cetyl-, Lauryl- und Myristyl-Alkohol und Gemische derselben). Vorzugsweise werden Polyethylenglykole mit Molekulargewichten von 3000 - 20 000 eingesetzt.

Der Auftrag der Polierschicht erfolgt im Anschluß an die Lackierung des Mikrogranulats. Die Poliermittel können entweder in fester oder gelöster Form verarbeitet werden.

Die Größe der Mikrokapseln ist für die gute Akzeptanz der daraus herzustellenden flüssigen oralen Arzneiform von großer Bedeutung. Werden zu große Mikrokapseln verabreicht, kann ein subjektives "Sandgefühl" im Mund nicht ausgeschlossen werden. Darüberhinaus kann eine zu große Korngröße zu verstärkter Sedimentation der Mikrokapseln im Dispersionsmedium führen. Aus diesem Grunde ist eine Größe der Mikrokapseln von 10-1 000 μm, vorzugsweise von 10-800 μm und ganz besonders bevorzugt von 100- 500 μm anzustreben.

Die hier beschriebenen erfindungsgemäßen Mikrokapseln führen einerseits zu einer ausgezeichneten Geschmacksmaskierung und andererseits zu der aus pharmakokinetischen Gründen geforderten sehr raschen Wirkstofffreigabe. Innerhalb von 15-30 min. können mindestens 70-80 % des verkapselten Wirkstoffes in Lösung gebracht werden. Diese Forderung wird sowohl für stark saure (pH 1) wie auch schwächer saure (pH 4,5) pH-Medien im in-vitro-Versuch erfüllt. Dieses Ergebnis repräsentiert die hervorragende Bioverfügbarkeit des erfindungsgemäß formulierten Wirkstoffs.

Überraschenderweise konnte gefunden werden, daß

- eine vollständige Geschmacksmaskierung sowie die zur Gewährleistung einer hohen Bioverfügbarkeit geforderte rasche Wirkstofffreigabe dann erreichbar ist, wenn der Wirkstoff mit den erfindungsgemäßen verwendeten Hilfsstoffen mikroverkapselt wird,

- als Wirkstoff-Substanz die Betain-Form (Basisform des Wirkstoffs) gesetzt wird.

- neben einer vollständigen Geschmacksmaskierung eine rasche Wirkstofffreisetzung dann gewährleistet ist, wenn die Basisform des Wirkstoffs, Ciprofloxacin-Betain, zur Mikroverkapselung eingesetzt wird. Eine rasche Löslichkeit kann bei Verwendung einer geeigneten Lackrezeptur und Lack-Auftragsmenge sowohl im stark sauren wie auch im schwach sauren Medium (pH 1 und 4,5) gewährleistet werden. Demgegenüber ist die Geschmacksmaskierung des Wirkstoffs, der in einer löslichen Salzform der Basisform des Wirkstoffs eingesetzt werden, nicht mit den Lackmengen erreichbar, die zur Geschmacksmaskierung der Basisform des Wirkstoffs aussreichen. Weiterhin ist die Freisetzung von z.B. Ciprofloxacin-HCl aus Mikrokapseln verzögert.

- die rasche Freisetzung des in der Basisform vorliegenden Wirkstoffs dann gewährleistet werden kann, wenn ein spezieller Lack verwendet wird, der sowohl wasserunlösliche bzw. quellbare wie auch wasserlösliche Komponenten im geeigneten Verhältnis enthält.

Weiterhin wurden gefunden, daß die Verwendung der Anhydratform der Basisform des Wirkstoffs geeignet ist, eine ausreichend schnelle Freisetzung des Wirkstoffs aus Mikrokaspseln sowohl im stark sauren wie auch im schwach sauren Milieu zu gewährleisten. Somit wird eine Steuerung der Wirkstofffreigabe über den Feuchtegehalt der Mikrokapseln möglich: es kann einerseits gewährleistet werden, daß die Mikrokapseln über eine gewünschte Zeit "dicht" bleiben und somit die Geschmacksabdeckung gegeben ist. Es ist aber auch sichergestellt, daß nach variabel wählbarer Zeit der Wirkstoff aus den Mikrokapseln freigesetzt und resorbiert werden kann.

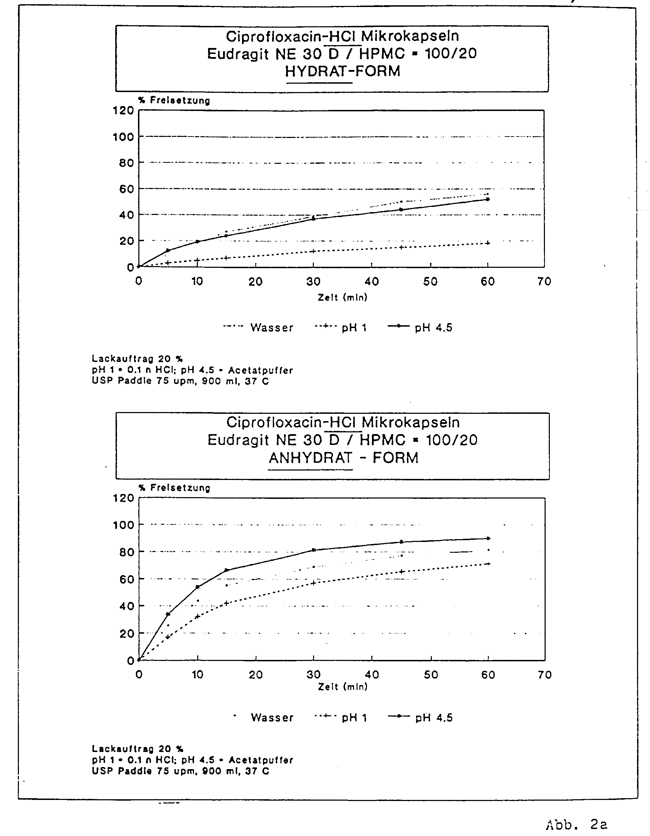

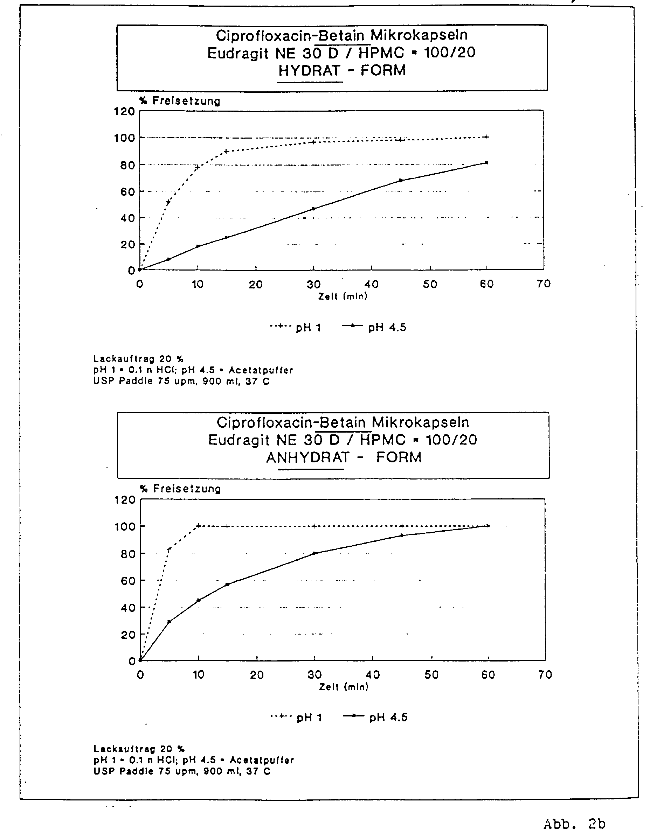

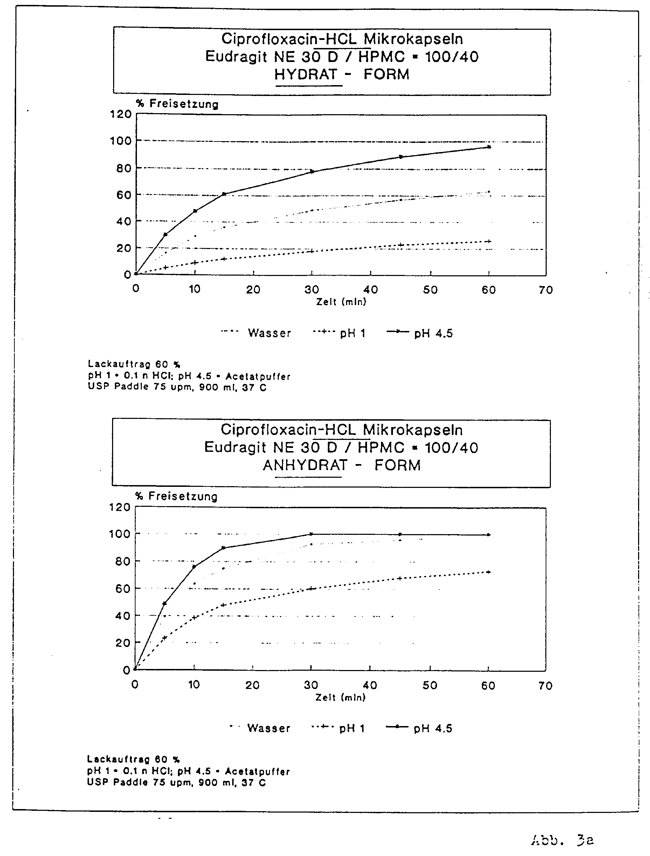

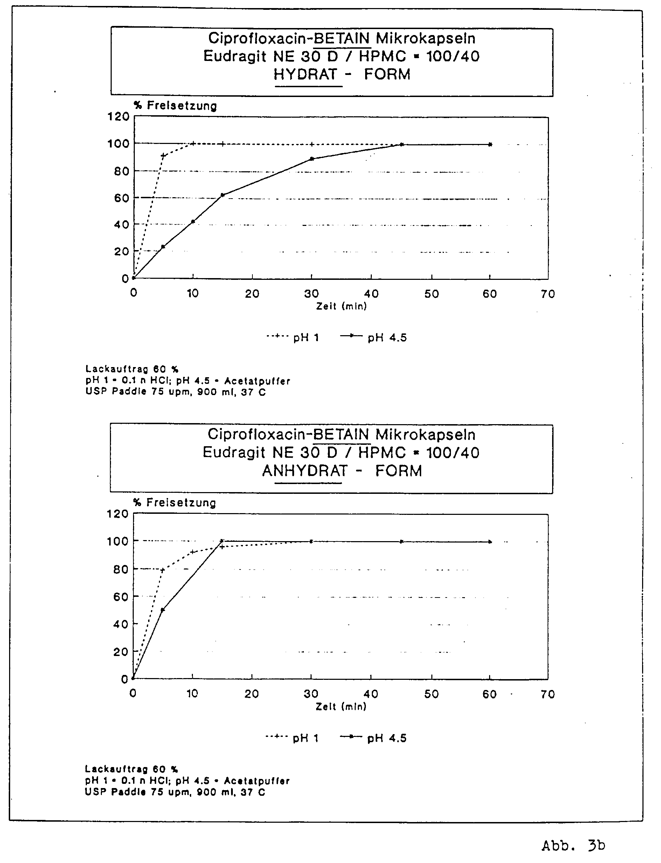

Die Verwendung der Mikrokapseln ist essentiell, wenn damit eine Saftförmulierung auf der Basis einer öligen Saftgrundlage gemäß der Erfindung, gefertigt werden soll, die den beschriebenen Freisetzungsanforderungen im stark bzw. schwach sauren Freisetzungsmilieu genügen soll (Abb. 2-5).

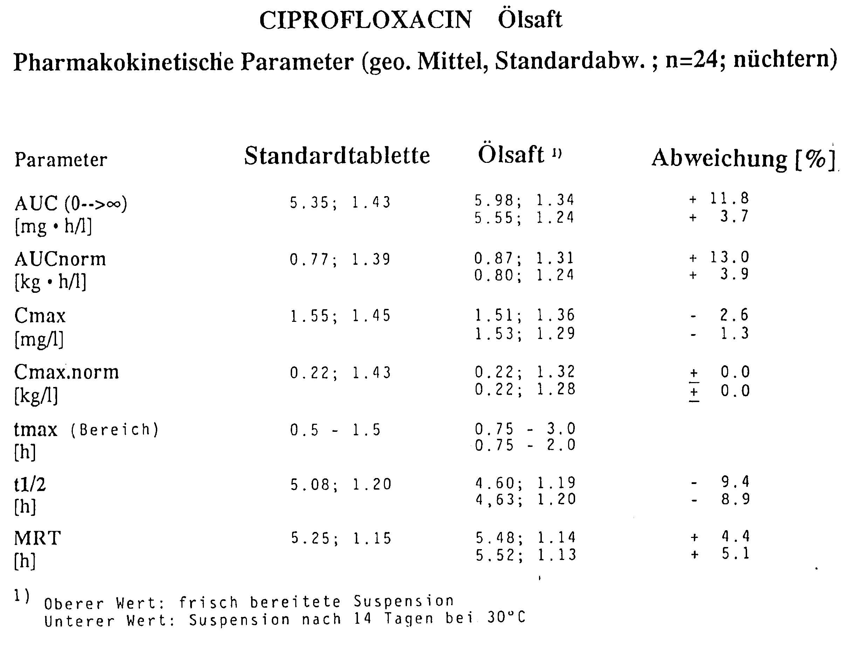

Die Bioverfügbarkeit einer repräsentativen erfindungsgemäßen Formulierungen entspricht der einer schnell freisetzenden Tablettenformulierung (Abb. 6).

Eine rasche Wirkstofffreisetzung kann entsprechend dem Stand der Technik auch durch die Verwendung eines Sprengmittels erreicht werden. Von Nachteil im Vergleich zu sprengmittelfreien Rezepturen ist jedoch, daß auf sprengmittelhaltige, den unangenehm schmeckenden Wirkstoff enthaltende Mikrokapselkerne erheblich höhere Lackmengen aufgetragen werden müssen, um eine geschmacksabdeckende Mikroverkapselung überhaupt zu gewährleisten. Dies führt zu einer Verlängerung der Mikroverkapselungsprozeßdauer und einer Erhöhung der Kosten.

Gegenstand der Erfindung ist auch die Steuerung der Freisetzungsgeschwindigkeit über den Feuchtegehalt der Mikrokapseln: durch Einstellung des Wassergehaltes z.B. der Ciprofloxacin-Mikrokapseln auf die Anhydrat-Stufe der Basisform des Wirkstoffs kann eine erhebliche Freisetzungsbeschleunigung bewirkt werden, die den für die Prozeßökonomie ungünstigen Einsatz eines üblichen Sprengmittels überflüssig macht.

Der Feuchtegehalt der Mikrokapseln ist auf unterschiedliche Art und Weise einstellbar:
So kann das zu überziehende Mikrogranulat vor dem Lackierprozeß auf einen gewünschten Feuchtegehalt eingestellt werden und nach dem Lackiervorgang gegebenenfalls einer zusätzlichen Nachtrocknung bis zur Anhydratstufe unterzogen werden.

Mikrogranulate, die ohne spezielle Vortrocknung lackiert werden und zunächst einen Wassergehalt von bis zu 30 Gew.-% aufweisen können, können durch eine Trocknung nach dem Mikroverkapselungsprozeß auf den gewünschten Wassergehalt (Anhydratstufe der Basisform des Wirkstoffs) eingestellt werden.

Saft auf öliger Basis (Mehrfachdosisform)

Die Bereitstellung einer wäßrigen Fertigsaftformulierung ist nicht möglich, da die Lackhülle der Mikrokapseln im wäßrigen Milieu nach einiger Zeit permeabel wird. Es ist daher von Vorteil, ein nichtwäßriges Dispersionsmedium für die Mikrokapseln zu wählen.

Geeignet sind ölige Dispersionsmedien wie Mandelöl, Arachisöl, Olivenöl, Mohnöl, Erdnußöl, Baumwollsamenöl, Sojabohnenöl, Maisöl, Ethyloleat, Oleyloleat, Isopropylmyristat und Isopropylpalmitat. Besonders geeignet sind Mittelkettige Triglyceride aufgrund ihres neutralen Geschmacks sowie ihrer günstigen Viskosität.

Als mit den genannten öligen Trägern kombinierbare flüssige Hilfsstoffe können Ethanol, Glycerol, Propylenglykol, Polyethylenglykol, 1,3-Butanol, Benzylalkohol, Diethylenglykol und Triethylenglykol u.a. verwendet werden.

Als weitere Zuschlagstoffe sind Netzmittel vorteilhaft einzusetzen. Ölige Saftformulierungen sind empfindlich gegen Feuchtigkeit. Bereits geringe Mengen an Wasser führen zu erheblichen Viskositätserhöhungen, die ein kontrolliertes Ausgießen der ursprünglich flüssigen Arzneiform aus dem Behältnis erschweren oder unmöglich machen können. Emulgatoren erhöhen einerseits die Wassertoleranz einer öligen Formulierung und erleichtern andererseits die Benetzbarkeit der Mikrokapseln beim Einarbeiten in die ölige Trägerflüssigkeit. Weiterhin verringern sie die Viskosität öliger Suspensionen. Als Netzmittel können die bereits beschriebenen Stoffe verwendet werden. Besonders vorteilhaft ist die Verarbeitung von Lecithin in Konzentrationen von 0,01 bis 20 %, ganz besonders vorteilhaft in Konzentrationen von 0,1-10 %, vorzugsweise von 0,5-5, %, bezogen auf eine Saftformulierung.

Weiterhin sind Kombinationen von Lecithin mit W/O-Emulgatoren wie z.B. Sorbitanfettsäureester-Typen, Fettalkoholen und Glycerolmono- und -di-Fettsäureestern besonders geeignet, die Empfindlichkeit von Ölsäften, die neben Wirkstoff-Mikrokapseln größere Mengen an Zuckern oder Zuckeraustauschstoffen enthalten, gegen Wasser herabzusetzen.

Als dichteerhöhende und damit suspensionsstabitisierende Zusätze sind Saccharose, Mannit, Sorbit, Xylit, Fructose, Glucose, Lactose und andere Zucker und Zuckeraustauschstoffe vorzugsweise geeignet. Die Konzentration im Ölsaft liegt bei 5-70 %, vorzugsweise bei 15-60 %, ganz besonders bei 20-40 %. Diese Stoffe müssen im Ölsaft in sehr feiner Teilchengröße (mittlere Teilchengröße ca. 1-50 µm, besonders bevorzugt 3-20 µm) vorliegen. Dies wird erreicht, indem entweder gemahlene Stoffe eingesetzt werden oder die ölige Suspensionsgrundlage durch Naßmahlung homogenisiert wird.

Überraschenderweise wurde gefunden, daß bei Verwendung von Saccharose die beste physikalische Stabilität für ölige Suspensionssäfte erreicht werden kann.

Als Antioxidantien zum Schutz öliger Trägermeden finden Stoffe wie $\alpha$-, $\beta$-, $\gamma$-, $\delta$-Tocopherol, Ascorbylpalmitat, Ascorbylstearat, L-Cystein, Thiodipropionsäure, Thiomilchsäure, Thioglycolsäure, Monothioglycerol, Propylgallat, Butylhydroxyanisol, Butylhydroxytoluol u.a Verwendung.

Als antimikrobielle Hilfsstoffe können Phenol, Kresol (o-, p, m-), p-Chlor-m-kresol, Benzylalkohol, Phenylethylalkohol, Phenoxyethylalkohol, Chlorbutanol, p-Hydroxybenzoesäure-methylester, -ethylester, -propylester, -butylester, Benzalkoniumchlorid und andere quarternäre Ammoniumverbindungen, Chlorhexidin-diacetat und -digluconat, Phenylquecksilber-Verbindungen, Thiomersal, Benzoesäure und ihre Salze, Sorbinsäure und ihre Salze, Ethanol, 1,2-Propylenglykol, Glycerin, 2-Brom-2-nitro-propan-1,3-diol, Cetrimid und 2,4,4'Trichlor-2'-hydroxydiphenylether in geeigneter Konzentration eingesetzt werden.

Zur Erhöhung der Sedimentationsstabilität können weiterhin viskositätserhöhende Stoffe wie kolloidale Kieselsäure, Bentonit etc. verwendet werden.

Weiterhin können Aromastoffe, Süß- und Farbstoffe zugesetzt werden.

Die Behältnisse, in die die Suspensionen, abgefüllt werden, können z.B. aus Glas oder aus Kunststoff bestehen. Dabei können die Behältermaterialien Substanzen enthalten, die dem Inhalt einen besonderen Schutz, z.B. einen Lichtschutz verleihen.

Die Zusammensetzung der für eine Ölsaftformulietung eingesetzten Mikrokapseln, insbesondere die Wirkstoffqualität und deren Hydratstufe sowie die Filmkomposition und Lackauftragsmenge, sind von entscheidender Bedeutung für die Qualität einer derartigen Zubereitung. Die geforderte rasche Wirkstofffreisetzung kann überraschenderweise dann erhalten werden, wenn die Basisform des Wirkstoffs in den im Ölsaft suspendierten Mikrokapseln als Anhydrat vorliegt; dagegen ergeben entsprechende Rezepturen mit Mikrokapseln, die einen höheren Wassergehalt, etwa in Höhe der Dihydratstufe aufwiesen, eine nicht akzeptable, zu langsame Wirkstofffreisetzung.

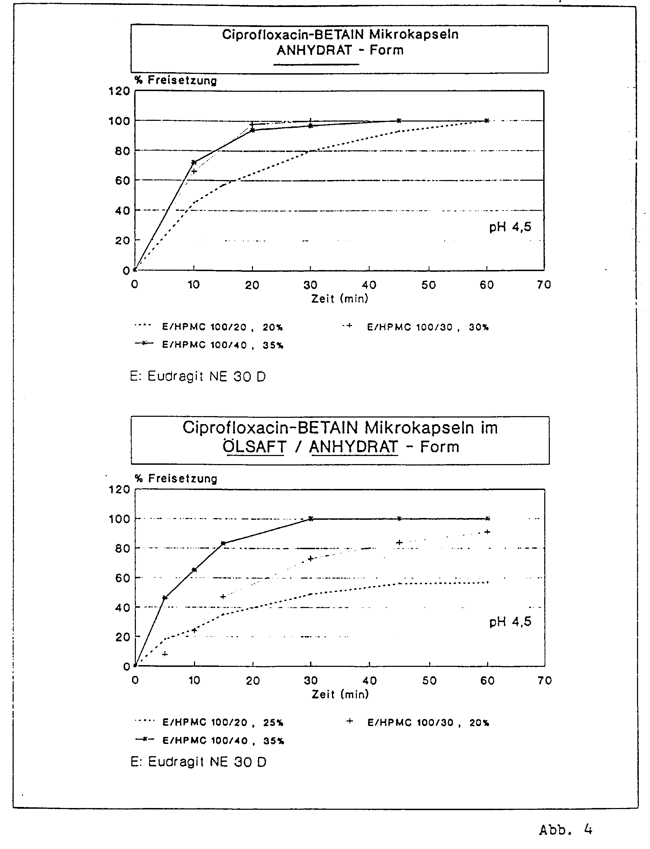

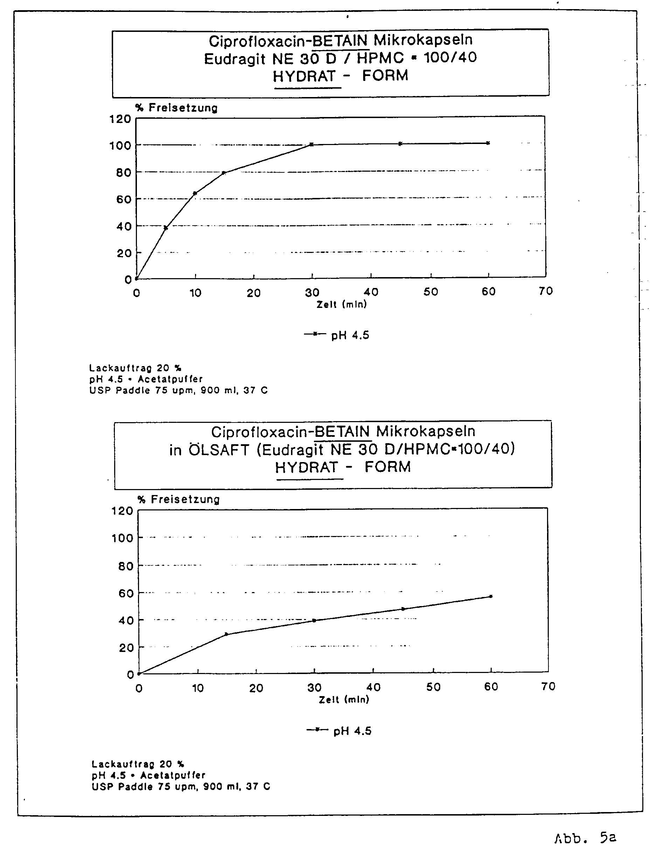

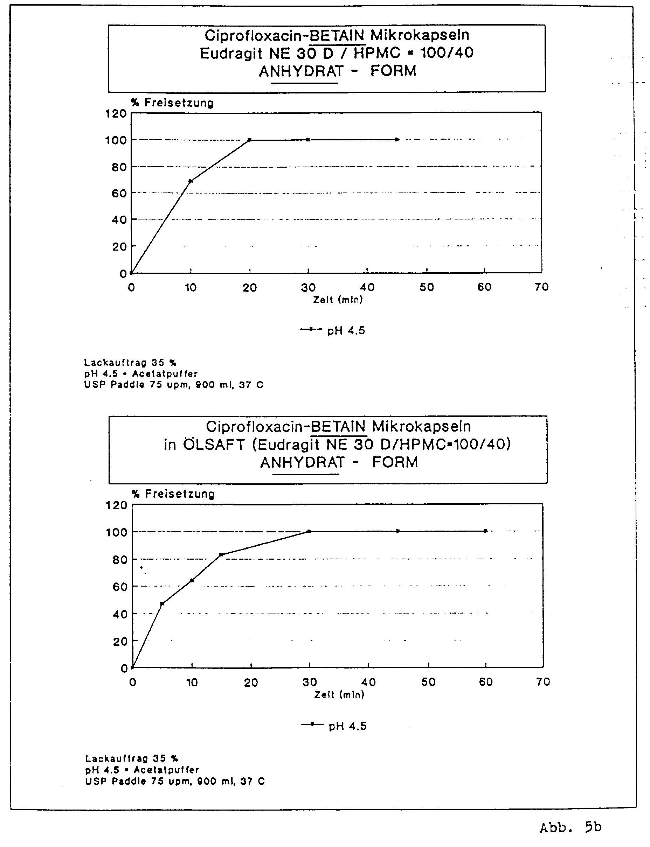

Es wurde weiterhin festgestellt, daß z.B. für Ciprofloxacin-Ölsäfte besonders solche Mikrokapseln geeignet sind, die ®Eudragit 12.5, ®Eudragit RL 30, ®Eudragit RS 30 D und/oder Ethylcellulose und z.B. HPMC und ebenso Magnesiumstearat oder Talcum als Lackkomponenten enthalten. Besonders bevorzugt sind jedoch Mikrokapseln, die eine Mischung von ®Eudragit NE 30 D und HPMC sowie Magnesiumstearat als Lackkomponenten enthalten: mit dieser Lackkomposition konnte überraschenderweise eine hinsichtlich der Geschmacksmaskierung stabile Ölsaftformulierung hergestellt werden. Als ganz besonders bevorzugte Lackzusammensetzung hinsichtlich Geschmacksmaskierung und Freisetzungseigenschaften sind Rezepturen mit ®Eudragit NE 30 D und HPMC im Verhältnis 100 zu 20 bis 50 Gew.-Teilen unter Zusatz von Magnesiumstearat und Tween 20 anzusprechen (Abb. 4,5), besonders bevorzugt ist ein Verhältnis von 100 zu 40 Gew.-Teilen.

Eine Saftformulierung auf öliger Basis kann in unterschiedlicher Form bereitgestellt werden: als Fertigsaft oder als "öliger Trockensaft". Ein öliger Fertigsaft besteht aus den im vorangegangenen genannten Saftkomponenten sowie dem darin suspendierten mikroverkapselten Wirkstoff. Die Stabilität der Formulierung muß über mehrere Jahre gewährleistet sein; insbesondere die Wirkstofffreisetzung darf auch während Lagerzeiten von mehreren Jahren keinen signifikanten Veränderungen unterliegen.

Unter einem "öligen Trockensaft" ist eine Formulierung zu verstehen, die aus einem öligen Placebosaft und den separat abgepackten Mikrokapseln besteht. Der Anwender stellt vor Gebrauch die applikationsfähige Arzneiform in der Weise her, daß er die separat abgepackten Mikrokapseln dem öligen Placebosaft hinzufügt.

Die Stabilität ist sowohl für den Placebosaft wie auch für die Mlkrokapseln, die getrennt verpackt vorliegen, separat zu gewährleisten. Weiterhin ist die Stabilität des daraus zubereiteten applikationsfertigen Verum-Ölsaftes über den Applikationszeitraum von z.B. 5-15 Tagen erforderlich. Die Zusammensetzung des zubereiteten "öligen Trockensaftes" ist in der Regel mit der des Fertigsaftes identisch.

Als Packmittel für die separat abgepackten Mikrokapseln können beispielsweise Glasflaschen oder Beutel aus geeigneten Kunststoff- oder Metallfolien verwendet werden. Das Beutelmaterial muß impermeabel für Wasser bzw. Wasserdampf sein, damit die Stabilität der in den Mikrokapseln enthaltenen Anhydrat-Form des Wirkstoffs sichergestellt ist.

| Zusammensetzungen von den Wirkstoff in Betain-Form enthaltenden Mikrogranulaten zur Herstellung von Mikrokapseln | | | |
|---|---|---|---|
| Beispiel | 1 mit PVP 25 | 2 ohne PVP 25 | 3 Vergleichsbeispiel mit Sprengmittel |
| Ciprofloxacin (-Betain) | 10,00 | 10,00 | 10,00 |
| PVP 25 | 0,70 | - | 0,70 |
| Ac-Di-Sol | - | - | 1,00 |
|  | 10,70 | 10,00 | 11,70 |
| Herstellung | Feuchtgranulation<br>Wirbelschichtsprühgranula-tion | Feuchtgranulation<br>- | Feuchtgranulation<br>Wirbelschichtsprühgranula-tion |

| Zusammensetzung von den Wirkstoff in Hydrochlorid-Form enthaltenden Mikrokapseln mit ®Eudragit NE 30 D / HPMC-Lacken (Vergleichsbeispiele) | | | |
|---|---|---|---|
| Beispiel | 4 | 5 | 6 |
| ®Eudragit NE 30 D / HPMC oder MC= | 100/10 | 100//20 | 100/20 |
| Lackauftragsmenge (theoret.) | 40% | 15% | 40% |
| Ciprofloxacin-HCl | 1470,00 | 1470,00 | 1470,00 |
| PVP 25 | 30,0 | 30,0 | 30,0 |
| ®Eudragit NE 30 D (Trockensubstanz) | 469,2 | 159,4 | 425,2 |
| HPMC 3 cp | - | 32,0 | 85,3 |
| Tylose MH 300 | 46,8 | - | - |
| Magnesiumstearat | 84,0 | 32,0 | 85,3 |
| Tween 20 | 4,2 | 1,6 | 4,2 |

| Zusammensetzung von den Wirkstoff in Hydrochlorid-Form enthaltenden Mikrokapseln mit [®]Eudragit NE 30 D / HPMC-Lacken (Vergleichsbeispiele) | | | | | |
|---|---|---|---|---|---|
| Beispiel | 7 | 8 | 9 | 10 | 11 |
| [®]Eudragit NE 30 D / HPMC oder MC= | 100/30 | 100/30 | 100/40 | 100/40 | 100/40 |
| Lackauftragsmenge (theoret.) | 20 % | 30 % | 50 % | 60 % | 80 % |
| Ciprofloxacin-HCl | 1470,00 | 1470,00 | 1470,00 | 1470,00 | 1470,00 |
| PVP 25 | 30,0 | 30,0 | 30,0 | 30,0 | 30,0 |
| [®]Eudragit NE 30 D (Trockensubstanz) | 201,9 | 302,9 | 468,75 | 562,5 | 750,0 |
| HPMC 3 cp | 60,7 | 91,0 | 187,50 | 225,0 | 300,0 |
| Magnesiumstearat | 35,4 | 53,1 | 93,75 | 112,5 | 150,0 |
| Tween 20 | 2,1 | 3,1 | 5,25 | 6,3 | 8,4 |

| Zusammensetzung von den Wirkstoff in Hydrochlorid-Form enthaltenden Mikrokapseln mit [®]Eudragit NE 30 D / HPMC-Lacken (Vergleichsbeispiele) | | |
|---|---|---|
| Beispiel | 12 | 13 |
| [®]Eudragit RL 30 D / Triethylcitrat= | 100/10 | 100/20 |
| Lackauftragsmenge (theoret.) | 50 % | 60 % |
| Ciprofloxacin-HCl | 1470,00 | 1470,00 |
| Maisstärke feucht | 30,00 | 30,00 |
| [®]Eudragit RL 30 D (Trockensubstanz) | 387,55 | 442,80 |
| Triethylcitrat | 39,00 | 88,65 |
| Talkum | 323,25 | 368,55 |

| Zusammensetzung von den Wirkstoff in Hydrochlorid-Form enthaltenden Mikrokapseln mit [®]Eudragit E 12,5 (Vergleichsbeispiele) | |
|---|---|
| Beispiel | 14 |
| Lackauftragsmenge (theoret.) | 50% |
| Ciprofloxacin-HCl | 1470,00 |
| Maisstärke feucht | 30,00 |
| [®]Eudragit E 12,5 (Trockensubstanz) | 409,50 |
| Mikrokristalline Cellulose | 170,25 |

(fortgesetzt)

| Zusammensetzung von den Wirkstoff in Hydrochlorid-Form enthaltenden Mikrokapseln mit ®Eudragit E 12,5 (Vergleichsbei- spiele) | |
|---|---|
| Beispiel | 14 |
| Magnesiumstearat | 170,25 |

| Zusammensetzung von den Wirkstoff in Betain-Form enthaltenden Mikrokapseln mit ®Eudragit NE 30 D / HPMC-Lacken | | | | | |
|---|---|---|---|---|---|
| Beispiel | 15 | 16 | 17 | 18 | 19 |
| ®Eudragit NE 30 D / HPMC= | 100/10 | 100/20 | 100/20 | 100/20 | 100/30 |
| Lackauftragsmenge (theoret.) | 20% | 15 % | 20% | 25% | 20% |
| Ciprofloxacin | 10,000 | 10,000 | 10,000 | 10,000 | 10,000 |
| PVP 25 | 0,700 | 0,700 | 0,700 | 0,700 | 0,700 |
| ®Eudragit NE 30 D (Trockensubstanz) | 1,660 | 1,138 | 1,517 | 1,897 | 1,440 |
| HPMC 3 cp | 0,167 | 0,228 | 0,304 | 0,380 | 0,432 |
| Magnesiumstearat | 0,298 | 0,228 | 0,304 | 0,380 | 0,252 |
| Tween 20 | 0,015 | 0,012 | 0,015 | 0,019 | 0,015 |

| Zusammensetzung von den Wirkstoff in Betain-Form enthaltenden Mikrokapseln mit ®Eudragit NE 30 D / HPMC-Lacken | | | | | |
|---|---|---|---|---|---|
| Beispiel | 20 | 21 | 22 | 23 | 24 |
| ®Eudragit NE 30 D / HPMC= | 100/40 | 100/40 | 100/40 | 100/40 | 100/40 |
| Lackauftragsmenge (theoret.) | 20% | 35% | 40% | 50% | 60% |
| Ciprofloxacin | 10,000 | 10,000 | 10,000 | 10,000 | 10,000 |
| PVP 25 | 0,700 | 0,700 | 0,700 | 0,700 | 0,700 |
| ®Eudragit NE 30 D (Trockensubstanz) | 1,328 | 2,324 | 2,656 | 3,320 | 3,984 |
| HPMC 3 cp | 0,531 | 0,929 | 1,062 | 1,328 | 1,593 |
| Magnesiumstearat | 0,265 | 0,464 | 0,530 | 0,662 | 0,795 |
| Tween 20 | 0,015 | 0,026 | 0,030 | 0,038 | 0,045 |

| Zusammensetzung von den Wirkstoff in Betain-Form enthaltenden Mikrokapseln mit ®Eudragit NE 30 D / HPMC-Lacken und Polierschicht | |
|---|---|
| Beispiel | 25 |
| ®Eudragit NE 30 D / HPMC= | 100/40 |
| Lackauftragsmenge (theoret.) | 20 % |
| Ciprofloxacin | 10,000 |
| PVP 25 | 0,700 |
| ®Eudragit NE 30 D (Trockensubstanz) | 1,328 |
| HPMC 3 cp | 0,531 |
| Magnesiumstearat | 0,265 |
| Tween 20 | 0,015 |
| PEG 6000 | 0,642 |

| Zusammensetzung von den Wirkstoff in Betain-Form enthaltenden Mikrokapseln mit ®Eudragit NE 30 D / HPMC-Lacken (Vergleichsbeispiele mit Sprengmittel-Zusatz) | | |
|---|---|---|
| Beispiel | 26 | 27 |
| ®Eudragit NE 30 D / HPMC= | 100/30 | 100/30 |
| Lackauftragsmenge (theoret.) | 60 % | 60 % |
| Ciprofloxacin | 10,000 | 10,000 |
| PVP 25 | 0,700 | 0,700 |
| Ac-Di-Sol | 1,000 | 1,000 |
| ®Eudragit NE 30 D (Trockensubstanz) | 4,725 | 4,725 |
| HPMC 3 cp | 1,419 | 1,419 |
| Magnesiumstearat | 0,828 | 0,828 |
| Tween 20 | 0,048 | 0,048 |
| PEG 6000 | - | 0,936 |

| Zusammensetzung von den Wirkstoff in Betain-Form enthaltenden Mikrokapseln mit ®Eudragit RL 30 D-Lacken | | |
|---|---|---|
| Beispiel | 28 | 29 |
| ®Eudragit RL 30 D / Triethylcitrat = | 100/20 | 100/10 |
| Lackauftragsmenge (theoret.) | 50% | 60% |
| Ciprofloxacin | 10,000 | 10,000 |

(fortgesetzt)

| Zusammensetzung von den Wirkstoff in Betain-Form enthaltenden Mikrokapseln mit ®Eudragit RL 30 D-Lacken | | |
|---|---|---|
| Beispiel | 28 | 29 |
| PVP 25 | 0,700 | 0,700 |
| ®Eudragit RL 30 D (Trockensubstanz) | 2,638 | 3,320 |
| Talkum gesiebt | 2,188 | 2,760 |
| Triethylcitrat | 0,525 | 0,340 |

| Zusammensetzung von den Wirkstoff in Betain-Form enthaltenden Mikrokapseln mit ®Eudragit RL 30 D-Lacken (Vergleichsbeispiele mit Sprengmittel-Zusatz) | | |
|---|---|---|
| Beispiel | 30 | 31 |
| ®Eudragit RL 30 D / Triethylcitrat= | 100/20 | 100/20 |
| Lackauftragsmenge (theoret.) | 60% | 60% |
| Ciprofloxacin | 10,000 | 10,000 |
| PVP 25 | 0,700 | 0,700 |
| Ac-Di-Sol | 1,000 | 1,000 |
| ®Eudragit RL 30 D (Trockensubstanz) | 3,460 | 3,460 |
| Talkum gesiebt | 2,870 | 2,870 |
| Triethylcitrat | 0,690 | 0,690 |
| PEG 6000 | - | 0,936 |

| Beispiel gemäß der Erfindung Ciprofloxacin Mikrokapseln in Ölsaft-Formulierung (Angaben zur Herstellung von 140 ml Ciprofloxacin Saft 5% m/v) | |
|---|---|
| Ciprofloxacin | 7,000 |
| Polyvinylpyrrolidon 25 | 0,490 |
| ®Eudragit NE 30 D | 2,257 |
| HPMC 3 cp | 0,910 |
| Magnesiumstearat | 0,455 |
| Tween 20 | 0,018 |
| Miglyol 812 | 98,214 |
| Saccharose mikrofein | 39,025 |
| Lipoid S 75 | 1,405 |

(fortgesetzt)

| Beispiel gemäß der Erfindung Ciprofloxacin Mikrokapseln in Ölsaft-Formulierung (Angaben zur Herstellung von 140 ml Ciprofloxacin Saft 5% m/v) | |
|---|---|
| Erdbeeraroma | 0,156 |
| Mikrokapseln: ®Eudragit NE 30 D / HPMC = 100/40; Lackauftrag 48,6 %. | |

**Patentansprüche**

1. Durch Mikroverkapselung geschmacksmaskierte Wirkstoffe enthaltende pharmazeutische Mittel mit schneller Wirkstoffreisetzung und hoher Bioverfügbarkeit, dadurch gekennzeichnet, daß der mikroverkapselte Wirkstoff Ciprofloxacin-Betain mit weniger als 5 %, insbesondere weniger als 3,0 % Wasser in Form von Kristallwasser oder sonstigen Wasser-Addukten ist und die Kapselwand aus einem Lack aus wasserunlöslichen neutralen Methyl- und/oder Ethyl-Ester-Verbindungen oder quartären Ammoniumverbindungen der Polymethacrylsäure oder Mischungen daraus oder Ethylcellulose besteht, der gegebenenfalls zusätzliche wasserlösliche Polymere, Weichmacher, Netzmittel und andere übliche Hilfsstoffe enthalten kann, und die pharmazeutischen Mittel als ölige Saftformulierung vorliegen.

2. Pharmazeutische Mittel gemäß Anspruch 1, dadurch gekennzeichnet, daß die Kapselwand aus einem Lack besteht, der als Filmbildner neutrale Methyl- und/oder Ethyl-Ester-Verbindungen der Polymethacrylsäure (®Eudragit NE 30 D, Röhm, Darmstadt), und/oder quartäre Ammoniumverbindungen der Polymethacrylsäure (®Eudragit RL 30 D, ®Eudragit RS 30 D) oder Ethylcellulose und Triethylcitrat und gegebenenfalls Hydroxypropylmethylcellulose enthält.

3. Pharmazeutische Mittel gemäß Ansprüchen 1 und 2, dadurch gekennzeichnet, daß die Kapselwand aus einem Lack enthaltend ein Gemisch aus 100 Gew.-Teilen neutraler Methyl- und/oder Ethyl-Ester-Verbindungen der Polymethacrylsäure (®Eudragit NE 30) und 20 bis 50 Gew.-Teilen Hydroxypropylmethylcellulose und gegebenenfalls üblichen Hilfsstoffen besteht.

4. Pharmazeutische Mittel gemäß Ansprüchen 1 bis 3, dadurch gekennzeichnet, daß die Mikrokapseln eine Größe von 10 bis 800 μm aufweisen.

5. Verfahren zur Herstellung von pharmazeutischen Mitteln gemäß Ansprüchen 1 bis 4, dadurch gekennzeichnet, daß das Ciprofloxacin-Betain feucht granuliert wird, anschließend gegebenenfalls durch Trocknung der Feuchtegehalt des Wirkstoffs auf 0 bis 30 Gew.-% eingestellt wird, das so erhaltene Mikrogranulat mit einem Lack auf Basis neutraler Methyl und/oder Ethyl-Esterverbindungen oder quartärer Ammoniumverbindungen der Polymethacrylsäure und Ethylcellulose, der gegebenenfalls wasserlösliche Polymere, Weichmacher, Netzmittel und andere übliche Hilfsstoffe enthalten kann, beschichtet wird, anschließend der gewünschte Feuchtegehalt des Wirkstoffs Ciprofloxacin-Betain gegebenenfalls durch Trocknung eingestellt wird, die so erhaltenen Mikrokapseln gegebenenfalls poliert werden und als Ölsaft formuliert werden.

**Claims**

1. Pharmaceutical compositions containing active ingredients which are flavour-masked by microencapsulation having rapid release of active ingredient and high bioavailability, characterized in that the microencapsulated active ingredient is ciprofloxacin-betaine containing less than 5%, in particular less than 3.0%, of water in the form of water of crystallization or other water adducts and the capsule wall consists of a coating of water-insoluble neutral methyl and/or ethyl ester compounds or quaternary ammonium compounds of polymethacrylic acid or mixtures thereof or ethylcellulose and which can optionally contain additional water-soluble polymers, plasticizers, wetting agents and other customary auxiliaries, and the pharmaceutical compositions are formulated as an oily juice.

2. Pharmaceutical compositions according to Claim 1, characterized in that the capsule wall consists of a coating which contains neutral methyl and/or ethyl ester compounds of polymethacrylic acid (®Eudragit NE 30 D, Röhm, Darmstadt), and/or quaternary ammonium compounds of polymethacrylic acid (®Eudragit RL 30 D, ®Eudragit RS 30 D) or ethylcellulose and triethyl citrate and, if appropriate, hydroxypropylmethylcellulose as film formers.

3. Pharmaceutical compositions according to Claims 1 and 2, characterized in that the capsule wall consists of a coating containing a mixture of 100 parts by weight of neutral methyl- and/or ethyl ester compounds of polymethacrylic acid (®Eudragit NE 30) and 20 to 50 parts by weight of hydroxypropylmethylcellulose and, if appropriate, customary auxiliaries.

4. Pharmaceutical compositions according to Claims 1 to 3, characterized in that the microcapsules have a size of 10 to 800 μm.

5. Processes for the preparation of pharmaceutical compositions according to Claims 1 to 4, characterized in that the ciprofloxacin-betaine is granulated in moist form, then the moisture content of the active ingredient is optionally adjusted by drying to 0 to 30% by weight, the microgranules thus obtained are coated with a coating based on neutral methyl and/or ethyl ester compounds or quaternary ammonium compounds of polymethacrylic acid and ethylcellulose, which coating can optionally contain water-soluble polymers, plasticizers, wetting agents and other customary auxiliaries, then the desired moisture content of the active ingredient ciprofloxacin-betaine is optionally adjusted by drying, the microcapsules thus obtained are optionally polished and are formulated as an oily juice.

**Revendications**

1. Compositions pharmaceutiques contenant des substances actives dont la saveur est masquée par microencapsulation, à libération rapide de substance active et à haute biodisponibilité, caractérisées en ce que la substance active micro-encapsulée est une bétaïne de ciprofloxacine avec moins de 5 %, notamment moins de 3,0 % d'eau sous forme d'eau de cristallisation ou d'autres produits d'addition d'eau et la paroi des capsules est constituée d'une laque de composés formés d'esters de méthyle et/ou d'éthyle ou de composés d'ammonium quaternaire d'acide polyméthacrylique neutres insolubles dans l'eau ou de mélanges de ces composés ou d'éthylcellulose, cette laque pouvant contenir éventuellement d'autres polymères, plastifiants, agents mouillants et autres substances auxiliaires classiques solubles dans l'eau, et les compositions pharmaceutiques étant formulées comme un sirop huileux.

2. Compositions pharmaceutiques suivant la revendication 1, caractérisées en ce que la paroi des capsules est constituée d'une laque qui contient comme agents filmogènes des composés formés d'esters de méthyle et/ou d'éthyle neutres d'acide polyméthacrylique (Eudragit® NE 30 D, Röhm, Darmstadt) et/ou des composés d'ammonium quaternaire d'acide polyméthacrylique (Eudragit® RL 30 D, Eudragit® RS 30 D) ou de l'éthylcellulose ou du citrate de triéthyle et, le cas échéant, de l'hydroxypropylméthylcellulose.

3. Compositions pharmaceutiques suivant les revendications 1 et 2, caractérisées en ce que la paroi des capsules est constituée d'une laque contenant un mélange de 100 parties en poids de composés formés d'esters de méthyle et/ou d'éthyle neutres d'acide polyméthacrylique (Eudragit® NE 30) et de 20 à 50 parties en poids d'hydroxypropylméthylcellulose et, le cas échéant, de substances auxiliaires classiques.

4. Compositions pharmaceutiques suivant les revendications 1 à 3, caractérisées en ce que les microcapsules ont un diamètre de 10 à 800 μm.

5. Procédé de préparation de compositions pharmaceutiques suivant les revendications 1 à 4, caractérisé en ce que la bétaïne de ciprofloxacine est granulée par voie humide, puis le cas échéant, la teneur en humidité de la substance active est ajustée à 0-30 % en poids par séchage, le produit microgranulé ainsi obtenu est revêtu d'une laque à base de composés formés d'esters de méthyle et/ou d'éthyle neutres ou de composés d'ammonium quaternaire d'acide polyméthacrylique et d'éthylcellulose, cette laque pouvant contenir, le cas échéant, des polymères, des plastifiants, des agents mouillants et d'autres substances auxiliaires classiques solubles dans l'eau, après quoi la teneur en humidité souhaitée de la substance active, c'est-à-dire de la bétaïne de ciprofloxacine, est ajustée, le cas échéant, par séchage, les microcapsules ainsi obtenues sont éventuellement lustrées et formulées comme un sirop huileux.

## In-Vitro-Freisetzung von CIPROFLOXACIN aus Mikrokapseln, hergestellt durch Sprüherstarrung in Carnaubawachs

% Freisetzung — 15 % m/m in Carnaubawachs

Zeit (min)

······ pH = 1      ──┼── pH = 4.5

Freisetzungsmethode: USP paddle 900 ml, 75 rpm
pH 1: 0.1 n HCl;  pH 4.5 : 0.05 m Acetatpuffer

Abb.1

**Ciprofloxacin-HCl Mikrokapseln**
**Eudragit NE 30 D / HPMC = 100/20**
**HYDRAT-FORM**

% Freisetzung

Zeit (min)

········ Wasser   ···+··· pH 1   ━•━ pH 4.5

Lackauftrag 20 %
pH 1 = 0.1 n HCl; pH 4.5 = Acetatpuffer
USP Paddle 75 upm, 900 ml, 37 C

**Ciprofloxacin-HCl Mikrokapseln**
**Eudragit NE 30 D / HPMC = 100/20**
**ANHYDRAT - FORM**

% Freisetzung

Zeit (min)

·   Wasser   ···+·· pH 1   ━•━ pH 4.5

Lackauftrag 20 %
pH 1 = 0.1 n HCl; pH 4.5 = Acetatpuffer
USP Paddle 75 upm, 900 ml, 37 C

Abb. 2a

**Ciprofloxacin-Betain Mikrokapseln**
**Eudragit NE 30 D / HPMC = 100/20**
**HYDRAT - FORM**

% Freisetzung

Zeit (min)

···+··· pH 1     —+— pH 4.5

Lackauftrag 20 %
pH 1 = 0.1 n HCl; pH 4.5 = Acetatpuffer
USP Paddle 75 upm, 900 ml, 37 C

**Ciprofloxacin-Betain Mikrokapseln**
**Eudragit NE 30 D / HPMC = 100/20**
**ANHYDRAT - FORM**

% Freisetzung

Zeit (min)

···+··· pH 1     —+— pH 4.5

Lackauftrag 20 %
pH 1 = 0.1 n HCl; pH 4.5 = Acetatpuffer
USP Paddle 75 upm, 900 ml, 37 C

Abb. 2b

## Ciprofloxacin-HCL Mikrokapseln
## Eudragit NE 30 D / HPMC = 100/40
## HYDRAT - FORM

% Freisetzung

Zeit (min)

··- Wasser    ··+·· pH 1    —•— pH 4.5

Lackauftrag 60 %
pH 1 = 0.1 n HCl; pH 4.5 = Acetatpuffer
USP Paddle 75 upm, 900 ml, 37 C

## Ciprofloxacin-HCL Mikrokapseln
## Eudragit NE 30 D / HPMC = 100/40
## ANHYDRAT - FORM

% Freisetzung

Zeit (min)

·· Wasser    ···+·· pH 1    —•— pH 4.5

Lackauftrag 60 %
pH 1 = 0.1 n HCl; pH 4.5 = Acetatpuffer
USP Paddle 75 upm, 900 ml, 37 C

Abb. 3a

Ciprofloxacin-BETAIN Mikrokapseln
Eudragit NE 30 D / HPMC = 100/40
HYDRAT - FORM

% Freisetzung

Lackauftrag 60 %
pH 1 = 0,1 n HCl; pH 4.5 = Acetatpuffer
USP Paddle 75 upm, 900 ml, 37 C

Ciprofloxacin-BETAIN Mikrokapseln
Eudragit NE 30 D / HPMC = 100/40
ANHYDRAT - FORM

% Freisetzung

Lackauftrag 60 %
pH 1 = 0,1 n HCl; pH 4.5 = Acetatpuffer
USP Paddle 75 upm, 900 ml, 37 C

Abb. 3b

### Ciprofloxacin-BETAIN Mikrokapseln
### ANHYDRAT - Form

% Freisetzung

pH 4,5

Zeit (min)

···· E/HPMC 100/20 , 20%   ·+ E/HPMC 100/30 , 30%

—*— E/HPMC 100/40 , 35%

E: Eudragit NE 30 D

### Ciprofloxacin-BETAIN Mikrokapseln im
### ÖLSAFT / ANHYDRAT - Form

% Freisetzung

pH 4,5

Zeit (min)

···· E/HPMC 100/20 , 25%   + E/HPMC 100/30 , 20%

—*— E/HPMC 100/40 , 35%

E: Eudragit NE 30 D

Abb. 4

Ciprofloxacin-BETAIN Mikrokapseln
Eudragit NE 30 D / HPMC = 100/40
HYDRAT - FORM

% Freisetzung

—■— pH 4.5

Lackauftrag 20 %
pH 4.5 · Acetatpuffer
USP Paddle 75 upm, 900 ml, 37 C

Ciprofloxacin-BETAIN Mikrokapseln
in ÖLSAFT (Eudragit NE 30 D/HPMC=100/40)
HYDRAT - FORM

% Freisetzung

—■— pH 4.5

Lackauftrag 20 %
pH 4.5 · Acetatpuffer
USP Paddle 75 upm, 900 ml, 37 C

Abb. 5a

Ciprofloxacin-BETAIN Mikrokapseln
Eudragit NE 30 D / HPMC = 100/40
ANHYDRAT - FORM

% Freisetzung

Lackauftrag 35 %
pH 4.5 = Acetatpuffer
USP Paddle 75 upm, 900 ml, 37 C

Ciprofloxacin-BETAIN Mikrokapseln
in ÖLSAFT (Eudragit NE 30 D/HPMC=100/40)
ANHYDRAT - FORM

% Freisetzung

Lackauftrag 35 %
pH 4.5 = Acetatpuffer
USP Paddle 75 upm, 900 ml, 37 C

Abb. 5b

# CIPROFLOXACIN   Ölsaft

## Pharmakokinetische Parameter (geo. Mittel, Standardabw. ; n=24; nüchtern)

| Parameter | Standardtablette | Ölsaft [1] | Abweichung [%] |
|---|---|---|---|
| AUC (0-->∞) [mg · h/l] | 5.35; 1.43 | 5.98; 1.34<br>5.55; 1.24 | + 11.8<br>+ 3.7 |
| AUCnorm [kg · h/l] | 0.77; 1.39 | 0.87; 1.31<br>0.80; 1.24 | + 13.0<br>+ 3.9 |
| Cmax [mg/l] | 1.55; 1.45 | 1.51; 1.36<br>1.53; 1.29 | - 2.6<br>- 1.3 |
| Cmax.norm [kg/l] | 0.22; 1.43 | 0.22; 1.32<br>0.22; 1.28 | ± 0.0<br>± 0.0 |
| tmax (Bereich) [h] | 0.5 - 1.5 | 0.75 - 3.0<br>0.75 - 2.0 | |
| t1/2 [h] | 5.08; 1.20 | 4.60; 1.19<br>4,63; 1.20 | - 9.4<br>- 8.9 |
| MRT [h] | 5.25; 1.15 | 5.48; 1.14<br>5.52; 1.13 | + 4.4<br>+ 5.1 |

1) Oberer Wert: frisch bereitete Suspension
   Unterer Wert: Suspension nach 14 Tagen bei 30°C

EP 0 551 820 B1